# EUROPEAN PATENT SPECIFICATION

(11) **EP 4 277 537 B1**
(45) Date of publication and mention of the grant of the patent: **11.03.2026**
(21) Application number: 22702164.9
(22) Date of filing: 14.01.2022
(51) Int. Cl.: A61B 8/12, A61B 8/00

(54) **INTRALUMINAL IMAGING DEVICE WITH THERMALLY BONDED IMAGING JOINT AND FLEXIBLE TRANSITION**
INTRALUMINALE BILDGEBUNGSVORRICHTUNG MIT THERMISCH VERBUNDENER BILDGEBUNGSVERBINDUNG UND FLEXIBLEM ÜBERGANG
DISPOSITIF D'IMAGERIE INTRACAVITAIRE AVEC JOINT D'IMAGERIE THERMOSOUDÉ ET TRANSITION FLEXIBLE

(30) Priority: 14.01.2021 US 202163137534 P
(43) Date of publication of application: 22.11.2023
(73) Proprietor: Philips Image Guided Therapy Corporation, San Diego CA 92130 (US); Koninklijke Philips N.V., 5656 AG Eindhoven (NL)
(72) Inventor: MINAS, Maritess, 5656 AG Eindhoven (NL)
(74) Representative: Philips Intellectual Property & Standards
(86) International application number: PCT/EP2022/050713
(87) International publication number: WO 2022/152827

(56) References cited:
- US-A1- 2005 215 942
- US-A1- 2019 307 420
- US-A1- 2020 054 304

## Description

### TECHNICAL FIELD

The present disclosure relates generally to intraluminal imaging devices and, in particular, to intraluminal imaging devices having flexible elongate members with reinforced heat-sealed joints. For example, intravascular ultrasound (IVUS) imaging catheter includes a flexible distal region that is heat sealed to an imaging assembly with the aid of a reinforcement member.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Phased array (also known as synthetic-aperture) IVUS catheters are a type of IVUS device commonly used today. Phased array IVUS catheters carry a scanner assembly that includes an array of ultrasound transducers positioned and distributed around its perimeter or circumference along with one or more integrated circuit controller chips mounted adjacent to the transducer array. The controllers select individual acoustic elements (or groups of elements) for transmitting a pulse of acoustic energy and for receiving the ultrasound echo signal corresponding to the transmitted ultrasound energy. By stepping through a sequence of transmit-receive pairs, the phased array IVUS system can synthesize the effect of a mechanically scanned ultrasound transducer but without moving parts (hence the solid-state designation). Since there is no rotating mechanical element, the transducer array can be placed in direct contact with the blood and vessel tissue with minimal risk of vessel trauma and without a need for an additional housing between the rotating element and the vessel lumen.

IVUS catheters must be stiff enough to be pushable, so that a clinician can advance them through the tortuous pathways of human vasculature without kinking the catheter. However, to facilitate navigation through these tortuous pathways, the catheters must also be flexible. It can be challenging to design IVUS catheters that meet both of these requirements simultaneously. In addition, the seal between the catheter body and the IVUS scanner assembly must resist the intrusion of body fluids, while presenting a joint that does not substantially increase the diameter of the device, beyond the diameter of the catheter and scanner assembly themselves. In current IVUS systems, forming the seal between the catheter and scanner assembly can be labor-intensive. US2020054304A1 describes intravascular imaging devices and their manufacturing where the devices include a flexible elongate member having a distal portion coupled to an imaging assembly surrounding a lumen. US2019307420A1 describes an imaging catheter including a flexible elongate member and a crown and crown sleeve for attaching a pull wire to the distal end of the felexible elongate member.

### SUMMARY

Disclosed herein is an intraluminal imaging device (e.g., an intravascular ultrasound or IVUS imaging device) advantageously providing both pushability and trackability for navigation through vasculature, along with a seal that does not substantially increase the diameter of the device, with reduced labor requirement for assembly. The seal can be fluid intrusion resistant. The device includes a flexible elongate member (e.g., a catheter) with a proximal portion and a distal portion, and an imaging assembly disposed at the distal portion for obtaining intraluminal image data. A guidewire lumen, defined by a polymer inner member, may extend from the proximal portion the distal portion of the flexible elongate member, in either a rapid-exchange or over-the-wire configuration. The flexible elongate member includes a polymer outer sheath or outer member. A distal end of the polymer outer sheath or outer member has a diameter larger than the diameter of the scanner assembly, so that the distal end fits over a proximal portion of the scanner assembly. The distal portion of the flexible elongate member, proximal to the imaging assembly, that includes a tube shaped (e.g., a cylindrical body surrounding a lumen) polymer filler member disposed outside the inner member defining the guidewire lumen and inside the outer member. When heated, the filler member thermally reflows and thermally bonds with the polymer inner member and polymer outer member, forming a seal that holds the outer member tight (e.g., water-tight) against the scanner assembly.

One general aspect of the intraluminal imaging device includes an intraluminal imaging device. The intraluminal imaging device includes a flexible elongate member configured to be positioned within a body lumen of a patient, where the flexible elongate member includes a first polymer; an ultrasound scanner assembly configured to obtain ultrasound imaging data while positioned within the body lumen, where the ultrasound scanner assembly is positioned at a distal end of the flexible elongate member; and a filler member including a second polymer and positioned at a distal end of the flexible elongate member, where the filler member is coupled to the flexible elongate member via thermal reflow of the first polymer and the second polymer to seal a joint between the flexible elongate member and the ultrasound scanner assembly. The filler member includes a lumen. The flexible elongate member includes: an outer member including a lumen; and an inner member extending within the lumen of the outer member and the lumen of the filler member. The outer member includes the first polymer, where the inner member includes a third polymer, and where the filler member is coupled to the flexible elongate member via thermal reflow of the second polymer and the third polymer.

Implementations may include one or more of the following features. In some embodiments, the inner member includes a lumen configured to receive a guidewire, and where the filler member is arranged such that, when the guidewire is received within the lumen of the inner member, the guidewire extends within the lumen of the filler member. In some embodiments, the filler member is positioned at a proximal portion of the ultrasound scanner assembly. In some embodiments, the distal end of the flexible elongate member is positioned over the proximal portion of the ultrasound scanner assembly and the filler member. In some embodiments, the distal end of the flexible elongate member includes a flared portion of the outer member, and where the flared portion is coupled to the filler member. In some embodiments, the ultrasound scanner assembly includes a support member and an array of acoustic elements positioned around the support member, and the filler member is positioned around a proximal portion of the support member. In some embodiments, the proximal portion of the support member includes a proximal flange defining a proximal end of the support member and configured to receive the inner member, and the filler member is positioned around the proximal flange. In some embodiments, the proximal portion of the support member includes a proximal stand, where a diameter of the proximal stand is greater than a diameter of the proximal flange, and where the filler member is positioned adjacent to the proximal stand. In some embodiments, the proximal portion of the support member terminates at a proximal end of the support member, and the proximal end of the filler member is positioned proximal of the proximal end of the support member. In some embodiments, the ultrasound scanner assembly includes a conductor interface, where the plurality of conductors are coupled to the conductor interface, and where the conductor interface is adjacent to at least one of the first polymer or the second polymer. In some embodiments, the plurality of conductors are coupled to the conductor interface at a proximal portion of the conductor interface, and the proximal portion of the conductor interface is positioned proximal of the filler member. In some embodiments, the flexible elongate member includes a rapid-exchange catheter with a guidewire entry port, where the guidewire entry port is disposed at a distal portion of the flexible elongate member, and where the filler member is positioned distal of the guidewire entry port.

One general aspect includes an intravascular ultrasound (IVUS) imaging catheter. The IVUS imaging catheter includes a catheter body configured to be positioned within a blood vessel of a patient, where the catheter body includes an outer member with a lumen and a first polymer; and an ultrasound scanner assembly including a circumferential array of acoustic elements configured to obtain ultrasound imaging data while positioned within the blood vessel, where the ultrasound scanner assembly is positioned at a distal end of the catheter body; and a filler member with a lumen and a second polymer, where the filler member is positioned at a distal end of the catheter body and a proximal portion of the ultrasound scanner assembly, where the distal end of the outer member is positioned around the proximal portion of the ultrasound scanner assembly and the filler member, where the catheter body further includes an inner member with a third polymer and extending within the lumen of the outer member and the lumen of the filler member, and where, to seal a joint between catheter body and the ultrasound scanner assembly, the filler member is coupled to the outer member via thermal reflow of the first polymer and coupled to the inner member via thermal reflow of the second polymer and the third polymer. Other embodiments of this aspect may include corresponding computer systems, apparatus, and computer programs recorded on one or more computer storage devices, each configured to perform the actions of the methods..

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
**Fig. 1A** is a diagrammatic schematic view of an intraluminal imaging system, according to aspects of the present disclosure.
**Fig. 1B** is a schematic diagram of a processor circuit, according to embodiments of the present disclosure.
**Fig. 2** is a diagrammatic view of the top of a scanner assembly in a flat configuration, according to aspects of the present disclosure.
**Fig. 3** is a diagrammatic perspective view of the scanner assembly shown in Fig. 2 in a rolled configuration around a support member, according to aspects of the present disclosure.
**Fig. 4** is a diagrammatic cross-sectional side view of a scanner assembly in a rolled configuration around a support member, according to aspects of the present disclosure.
**Fig. 5** is a side view of a distal portion of the intraluminal imaging device, including the flexible substrate and the outer member with an adhesive joint between them.
**Fig. 6** is a side view of a distal portion of the intraluminal imaging device, including the flexible substrate and the outer member with a heat seal joint between them, in accordance with at least one embodiment of the present disclosure.
**Fig. 7** is a flow diagram of an example assembly process for the heat seal joint as described in Fig. 6, in accordance with at least one embodiment of the present disclosure.
**Fig. 8A** is a side view of an intraluminal imaging device during an assembly process, in accordance with at least one embodiment of the present disclosure.
**Fig. 8B** is a side view of an intraluminal imaging device during an assembly process, in accordance with at least one embodiment of the present disclosure.
**Fig. 8C** is a side view of an intraluminal imaging device during an assembly process, in accordance with at least one embodiment of the present disclosure.
**Fig. 9** is a side cross-sectional view of an intraluminal imaging device prior to the formation of the heat seal joint, in accordance with at least one embodiment of the present disclosure.
**Fig. 10** is a cross-sectional end view, cut along plane 10-10 of Fig. 9, of the overlap region of an intraluminal imaging device prior to the formation of the heat seal joint, in accordance with at least one embodiment of the present disclosure.
**Fig. 11** is a side cross-sectional view of an intraluminal imaging device subsequent to the formation of the heat seal joint, in accordance with at least one embodiment of the present disclosure.
**Fig. 12** is a cross-sectional end view, cut along plane 12-12 of Fig. 11, of the heat seal region of an intraluminal imaging device subsequent to the formation of the heat seal joint, in accordance with at least one embodiment of the present disclosure.
**Fig. 13A** is a diagrammatic, schematic view of an intraluminal imaging device including a heat seal joint and a flexible elongate member.
**Fig. 13B** is a diagrammatic schematic view of an example intraluminal imaging device, where the flexible elongate member is implemented as an over-the-wire catheter.

### DETAILED DESCRIPTION

Disclosed herein is an intraluminal imaging device (e.g., an intravascular ultrasound or IVUS imaging device) advantageously providing both pushability and flexibility for navigation through vasculature, along with a seal that does not substantially increase the diameter of the device, and which requires less labor for assembly than existing intraluminal imaging device designs. The seal can be fluid ingress resistant. The intraluminal imaging device comprises a flexible elongate member (e.g., a catheter) for use within a body lumen (e.g., a blood vessel) of a patient. The flexible elongate member has a proximal portion and a distal portion, and an imaging assembly located at the distal portion and configured to obtain intraluminal image data (e.g., IVUS image data). A guidewire lumen, defined by a polymer inner member, may extend from the proximal portion the distal portion of the flexible elongate member, in either a rapid-exchange or over-the-wire configuration. The flexible elongate member includes a polymer outer sheath or outer member. A distal end of the polymer outer sheath or outer member has a diameter larger than the diameter of the scanner assembly, so that the distal end fits over a proximal portion of the scanner assembly. The distal portion of the flexible elongate member, proximal to the imaging assembly, that includes a tube shaped (e.g., a cylindrical body surrounding a lumen) polymer filler member disposed outside the inner member defining the guidewire lumen and inside the outer member. When heated, the filler member thermally reflows and thermally bonds with the polymer inner member and polymer outer member, forming a seal that holds the outer member tight (e.g., water-tight) against the scanner assembly.

The imaging assembly of the device includes a circumferential array of acoustic elements for gathering intravascular ultrasound (IVUS) image data. A distal portion of the inner member and a proximal portion of the imaging assembly are positioned within a polymer tube, which extends over a portion of a flexible substrate of the imaging assembly (e.g., over one or more weld legs that join the communication lines to the flexible substrate and/or over a plurality of integrated circuits on the flexible substrate). The flexible substrate is wrapped around a support member (e.g., a metallic ferrule). An insulating material (which may be an adhesive) is positioned around the support member and configured to prevent electrical contact (e.g., shorting) between the proximal portion of the flexible substrate and the support member. The inner member comprises a guidewire lumen extending from the proximal portion the distal portion of the flexible elongate member.

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

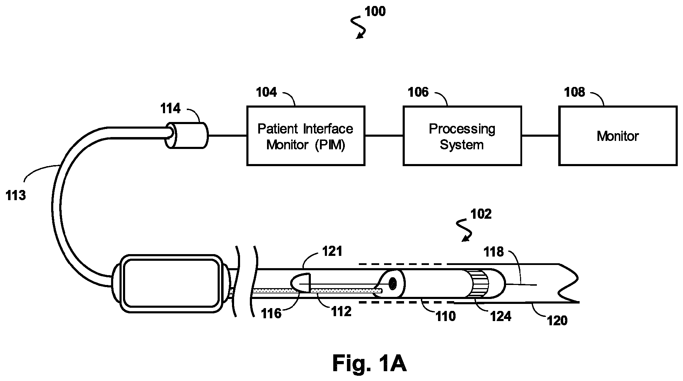

**Fig. 1A** is a diagrammatic schematic view of an ultrasound imaging system 100, according to aspects of the present disclosure. The ultrasound imaging system 100 can be an intraluminal imaging system. In some instances, the system 100 can be an intravascular ultrasound (IVUS) imaging system. The system 100 may include an intraluminal imaging device 102 such as a catheter, guide wire, or guide catheter, a patient interface module (PIM) 104, a processing system or console 106, and a monitor 108. The intraluminal imaging device 102 can be an ultrasound imaging device. In some instances, the device 102 can be IVUS imaging device, such as a solid-state IVUS device.

At a high level, the IVUS device 102 emits ultrasonic energy, or ultrasound signals, from a transducer array 124 included in scanner assembly 110 mounted near a distal end of the catheter device. The ultrasonic energy is reflected by tissue structures in the medium, such as a vessel 120, or another body lumen surrounding the scanner assembly 110, and the ultrasound echo signals are received by the transducer array 124. In that regard, the device 102 can be sized, shaped, or otherwise configured to be positioned within the body lumen of a patient. The PIM 104 transfers the received echo signals to the console or computer 106 where the ultrasound image (possibly including flow information) is reconstructed and displayed on the monitor 108. The processing system 106 can include a processor and a memory. The processing system 106 can be operable to facilitate the features of the IVUS imaging system 100 described herein. For example, the processing system 106 can execute computer readable instructions stored on the non-transitory tangible computer readable medium.

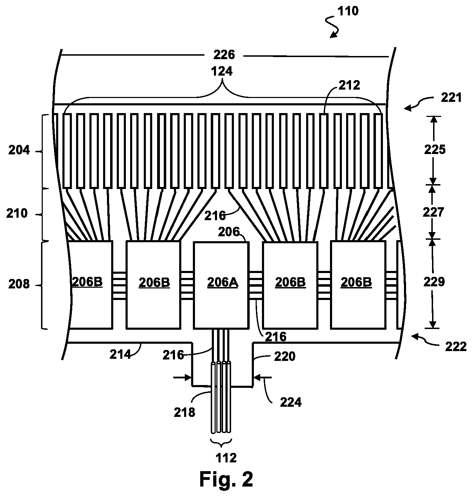

The PIM 104 facilitates communication of signals between the processing system 106 and the scanner assembly 110 included in the IVUS device 102. This communication includes the steps of: (1) providing commands to integrated circuit controller chip(s) 206A, 206B, illustrated in Fig. 2, included in the scanner assembly 110 to select the particular transducer array element(s), or acoustic element(s), to be used for transmit and receive, (2) providing the transmit trigger signals to the integrated circuit controller chip(s) 206A, 206B included in the scanner assembly 110 to activate the transmitter circuitry to generate an electrical pulse to excite the selected transducer array element(s) 212, and/or (3) accepting amplified echo signals received from the selected transducer array element(s) 212 via amplifiers included on the integrated circuit controller chip(s) 206 of the scanner assembly 110. In some embodiments, the PIM 104 performs preliminary processing of the echo data prior to relaying the data to the console 106. In examples of such embodiments, the PIM 104 performs amplification, filtering, and/or aggregating of the data. In an embodiment, the PIM 104 also supplies high- and low-voltage DC power to support operation of the device 102 including circuitry within the scanner assembly 110.

The processing system 106 receives the echo data from the scanner assembly 110 by way of the PIM 104 and processes the data to reconstruct an image of the tissue structures in the medium surrounding the scanner assembly 110. The console 106 outputs image data such that an image of the vessel 120, such as a cross-sectional image of the vessel 120, is displayed on the monitor 108. Vessel 120 may represent fluid filled or surrounded structures, both natural and man-made. The vessel 120 may be within a body of a patient. The vessel 120 may be a blood vessel, as an artery or a vein of a patient's vascular system, including cardiac vasculature, peripheral vasculature, neural vasculature, renal vasculature, and/or or any other suitable lumen inside the body. For example, the device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. In addition to natural structures, the device 102 may be used to examine man-made structures such as, but without limitation, heart valves, stents, shunts, filters and other devices.

In some embodiments, the IVUS device includes some features similar to traditional solid-state IVUS catheters, such as the EagleEye^{®} catheter available from Koninklijke Philips N.V. and those disclosed in U.S. Patent No. 7,846,101. For example, the IVUS device 102 includes the scanner assembly 110 near a distal end of the device 102 and a transmission line bundle 112 extending along the longitudinal body of the device 102. The transmission line bundle or cable 112 can include a plurality of conductors, including one, two, three, four, five, six, seven, or more conductors 218 (Fig. 2). Further, in some embodiments, the IVUS device 102 includes a plurality of transmission line bundles each comprising a plurality of conductors of varying size (e.g., gauge), insulation, and/or other structural and electrical characteristics. It is understood that any suitable gauge wire can be used for the conductors 218. In an embodiment, the cable 112 can include a four-conductor transmission line arrangement with, e.g., 41 AWG gauge wires. In an embodiment, the cable 112 can include a seven-conductor transmission line arrangement utilizing, e.g., 44 AWG gauge wires. In some embodiments, 43 AWG gauge wires can be used.

The transmission line bundle 112 passes through or connects to a cable 113 that terminates in a PIM connector 114 at a proximal end of the device 102. The PIM connector 114 electrically couples the transmission line bundle 112 to the PIM 104 and physically couples the IVUS device 102 to the PIM 104. In an embodiment, the IVUS device 102 further includes a guide wire exit port 116. Accordingly, in some instances the IVUS device is a rapid-exchange catheter. The guide wire exit port 116 allows a guide wire 118 to be inserted towards the distal end in order to direct the device 102 through the vessel 120.

In an embodiment, the processing system 106 generates flow data by processing the echo signals from the IVUS device 102 into Doppler power or velocity information. The processing system 106 may also generate B-mode data by applying envelope detection and logarithmic compression on the conditioned echo signals. The processing system 106 can further generate images in various views, such as 2D and/or 3D views, based on the flow data or the B-mode data. The processing system 106 can also perform various analyses and/or assessments. For example, the processing system 106 can apply virtual histology (VH) techniques, for example, to analyze or assess plaques within a vessel (e.g., the vessel 120). The images can be generated to display a reconstructed color-coded tissue map of plaque composition superimposed on a cross-sectional view of the vessel.

In an embodiment, the processing system 106 can apply a blood flow detection algorithm to determine the movement of blood flow, for example, by acquiring image data of a target region (e.g., the vessel 120) repeatedly and determining the movement of the blood flow from the image data. The blood flow detection algorithm operates based on the principle that signals measured from vascular tissue are relatively static from acquisition to acquisition, whereas signals measured from blood flow vary at a characteristic rate corresponding to the flow rate. As such, the blood flow detection algorithm may determine movements of blood flow based on variations in signals measured from the target region between repeated acquisitions. To acquire the image data repeatedly, the processing system 106 may control to the device 102 to transmit repeated pulses on the same aperture.

While the present disclosure describes embodiments related to intravascular ultrasound (IVUS) imaging using an intravascular catheter or guidewire, it is understood that one or more aspects of the present disclosure can be implemented in any suitable ultrasound imaging system, including a synthetic aperture ultrasound imaging system, a phased array ultrasound imaging system, or any other array-based ultrasound imaging system. For example, aspects of the present disclosure can be implemented in intraluminal ultrasound imaging systems using an intracardiac (ICE) echocardiography catheter and/or a transesophageal echocardiography (TEE) probe, and/or external ultrasound imaging system using an ultrasound probe configured for imaging while positioned adjacent to and/or in contact with the patient's skin. The ultrasound imaging device can be a transthoracic echocardiography (TTE) imaging device in some embodiments.

An ultrasound transducer array of the ultrasound imaging device includes an array of acoustic elements configured to emit ultrasound energy and receive echoes corresponding to the emitted ultrasound energy. In some instances, the array may include any number of ultrasound transducer elements. For example, the array can include between 2 acoustic elements and 10000 acoustic elements, including values such as 2 acoustic elements, 4 acoustic elements, acoustic elements, 64 acoustic elements, 128 acoustic elements, 500 acoustic elements, 812 acoustic elements, 3000 acoustic elements, 9000 acoustic elements, and/or other values both larger and smaller. In some instances, the transducer elements of the array may be arranged in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of transducer elements (e.g., one or more rows, one or more columns, and/or one or more orientations) can be uniformly or independently controlled and activated. The array can be configured to obtain one-dimensional, two-dimensional, and/or three-dimensional images of patient anatomy.

The ultrasound transducer elements may include piezoelectric/piezoresistive elements, piezoelectric micromachined ultrasound transducer (PMUT) elements, capacitive micromachined ultrasound transducer (CMUT) elements, and/or any other suitable type of ultrasound transducer elements. The ultrasound transducer elements of the array are in communication with (e.g., electrically coupled to) electronic circuitry. For example, the electronic circuitry can include one or more transducer control logic dies. The electronic circuitry can include one or more integrated circuits (IC), such as application specific integrated circuits (ASICs). In some embodiments, one or more of the ICs can include a microbeamformer (µBF). In other embodiments, one or more of the ICs includes a multiplexer circuit (MUX).

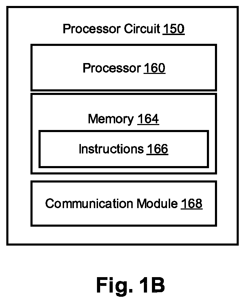

**Fig. 1B** is a schematic diagram of a processor circuit 150, according to embodiments of the present disclosure. The processor circuit 150 may be implemented in the processing system 106 and/or the imaging device 102 of Fig. 1A. As shown, the processor circuit 150 may include a processor 160, a memory 164, and a communication module 168. These elements may be in direct or indirect communication with each other, for example via one or more buses.

The processor 160 may include a central processing unit (CPU), a digital signal processor (DSP), an ASIC, a controller, an FPGA, another hardware device, a firmware device, or any combination thereof configured to perform the operations described herein. The processor 160 may also be implemented as a combination of computing devices, e.g., a combination of a DSP and a microprocessor, a plurality of microprocessors, one or more microprocessors in conjunction with a DSP core, or any other such configuration.

The memory 164 may include a cache memory (e.g., a cache memory of the processor 160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 164 includes a non-transitory computer-readable medium. The memory 164 may store instructions 166. The instructions 166 may include instructions that, when executed by the processor 160, cause the processor 160 to perform the operations described herein with reference to the processing system 106 and/or the imaging device 102 (Fig. 1A). Instructions 166 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 168 can include any electronic circuitry and/or logic circuitry to facilitate direct or indirect communication of data between the processor circuit 150, the imaging device 102, and/or the display 108. In that regard, the communication module 168 can be an input/output (I/O) device. In some instances, the communication module 168 facilitates direct or indirect communication between various elements of the processor circuit 150 and/or the processing system 106 (Fig. 1A).

**Fig. 2** is a diagrammatic top view of a portion of a flexible assembly 200, according to aspects of the present disclosure. The flexible assembly 200 includes a transducer array 124 formed in a transducer region 204 and transducer control logic dies 206 (including dies 206A and 206B) formed in a control region 208, with a transition region 210 disposed therebetween.

The transducer control logic dies 206 are mounted on a flexible substrate 214 into which the transducers 212 have been previously integrated. The flexible substrate 214 is shown in a flat configuration in Fig. 2. Though six control logic dies 206 are shown in Fig. 2, any number of control logic dies 206 may be used. For example, one, two, three, four, five, six, seven, eight, nine, ten, or more control logic dies 206 may be used.

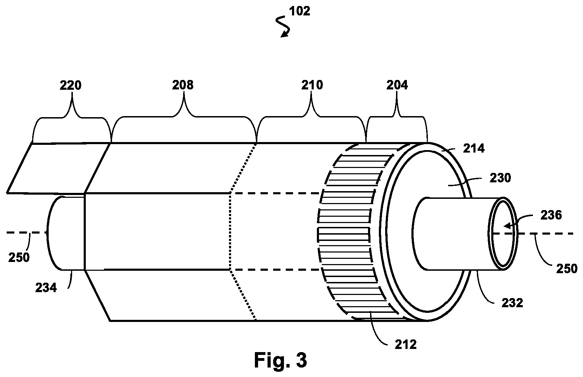

The flexible substrate 214, on which the transducer control logic dies 206 and the transducers 212 are mounted, provides structural support and interconnects for electrical coupling. The flexible substrate 214 may be constructed to include a film layer of a flexible polyimide material such as KAPTON^{™} (trademark of DuPont). Other suitable materials include polyester films, polyimide films, polyethylene napthalate films, or polyetherimide films, liquid crystal polymer, other flexible printed semiconductor substrates as well as products such as Upilex^{®} (registered trademark of Ube Industries) and TEFLON^{®} (registered trademark of E.I. du Pont). In the flat configuration illustrated in Fig. 2, the flexible substrate 214 has a generally rectangular shape. As shown and described herein, the flexible substrate 214 is configured to be wrapped around a support member 230 (Fig. 3) in some instances. Therefore, the thickness of the film layer of the flexible substrate 214 is generally related to the degree of curvature in the final assembled flexible assembly 200. In some embodiments, the film layer is between 5 µm and 100 µm, with some particular embodiments being between 5 µm and 25.1 µm, e.g., 6 µm.

The transducer control logic dies 206 is a non-limiting example of a control circuit. The transducer region 204 is disposed at a distal portion 221 of the flexible substrate 214. The control region 208 is disposed at a proximal portion 222 of the flexible substrate 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or, the length 227 of the transition region 210 may be less than lengths 225 and 229, the length 227 of the transition region 210 can be greater than lengths 225, 229 of the transducer region and controller region, respectively.

The control logic dies 206 are not necessarily homogenous. In some embodiments, a single controller is designated a master control logic die 206A and contains the communication interface for the transmission line bundle 112, which may serve as an electrical communication bus between a processing system, e.g., processing system 106, and the flexible assembly 200. Accordingly, the master control circuit may include control logic that decodes control signals received over the transmission line bundle 112, transmits control responses over the transmission line bundle 112, amplifies echo signals, and/or transmits the echo signals over the transmission line bundle 112. The remaining controllers are slave controllers 206B. The slave controllers 206B may include control logic that drives a transducer 212 to emit an ultrasonic signal and selects a transducer 212 to receive an echo. In the depicted embodiment, the master controller 206A does not directly control any transducers 212. In other embodiments, the master controller 206A drives the same number of transducers 212 as the slave controllers 206B or drives a reduced set of transducers 212 as compared to the slave controllers 206B. In an exemplary embodiment, a single master controller 206A and eight slave controllers 206B are provided with eight transducers assigned to each slave controller 206B.

To electrically interconnect the control logic dies 206 and the transducers 212, in an embodiment, the flexible substrate 214 includes conductive traces 216 formed in the film layer that carry signals between the control logic dies 206 and the transducers 212. In particular, the conductive traces 216 providing communication between the control logic dies 206 and the transducers 212 extend along the flexible substrate 214 within the transition region 210. In some instances, the conductive traces 216 can also facilitate electrical communication between the master controller 206A and the slave controllers 206B. The conductive traces 216 can also provide a set of conductive pads that contact the conductors 218 of the transmission line bundle 112 when the conductors 218 of the transmission line bundle 112 are mechanically and electrically coupled to the flexible substrate 214. Suitable materials for the conductive traces 216 include copper, gold, aluminum, silver, tantalum, nickel, and tin, and may be deposited on the flexible substrate 214 by processes such as sputtering, plating, and etching. In an embodiment, the flexible substrate 214 includes a chromium adhesion layer. The width and thickness of the conductive traces 216 are selected to provide proper conductivity and resilience when the flexible substrate 214 is rolled. In that regard, an exemplary range for the thickness of a conductive trace 216 and/or conductive pad is between 1-5 µm. For example, in an embodiment, 5 µm conductive traces 216 are separated by 5 µm of space. The width of a conductive trace 216 on the flexible substrate may be further determined by the width of the conductor 218 to be coupled to the trace/pad.

The flexible substrate 214 can include a conductor interface 220 in some embodiments. The conductor interface 220 can be a location of the flexible substrate 214 where the conductors 218 of the transmission line bundle 112 are coupled to the flexible substrate 214. For example, the bare conductors of the transmission line bundle 112 are electrically coupled to the flexible substrate 214 at the conductor interface 220. The conductor interface 220 can be a tab extending from the main body of flexible substrate 214. In that regard, the main body of the flexible substrate 214 can refer collectively to the transducer region 204, controller region 208, and the transition region 210. In the illustrated embodiment, the conductor interface 220 extends from the proximal portion 222 of the flexible substrate 214. In other embodiments, the conductor interface 220 is positioned at other parts of the flexible substrate 214, such as the distal portion 221, or the flexible substrate 214 may lack the conductor interface 220. A value of a dimension of the tab or conductor interface 220, such as a width 224, can be less than the value of a dimension of the main body of the flexible substrate 214, such as a width 226. In some embodiments, the substrate forming the conductor interface 220 is made of the same material(s) and/or is similarly flexible as the flexible substrate 214. In other embodiments, the conductor interface 220 is made of different materials and/or is comparatively more rigid than the flexible substrate 214. For example, the conductor interface 220 can be made of a plastic, thermoplastic, polymer, hard polymer, etc., including polyoxymethylene (e.g., DELRIN^{®}), polyether ether ketone (PEEK), nylon, Liquid Crystal Polymer (LCP), and/or other suitable materials.

**Fig. 3** illustrates a perspective view of the device 102 with the scanner assembly 200 in a rolled configuration. In some instances, the assembly 200 is transitioned from a flat configuration (Fig. 2) to a rolled or more cylindrical configuration (Fig. 3). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND SENSING ASSEMBLY HAVING A FLEXIBLE SUBSTRATE".

In some embodiments, the transducer elements 212 and/or the controllers 206 can be positioned in in an annular configuration, such as a circular configuration or in a polygon configuration, around a longitudinal axis 250 of a support member 230. It will be understood that the longitudinal axis 250 of the support member 230 may also be referred to as the longitudinal axis of the scanner assembly 200, the flexible elongate member 121, and/or the device 102. For example, a cross-sectional profile of the imaging assembly 200 at the transducer elements 212 and/or the controllers 206 can be a circle or a polygon. Any suitable annular polygon shape can be implemented, such as a based on the number of controllers/transducers, flexibility of the controllers/transducers, etc., including a pentagon, hexagon, heptagon, octagon, nonagon, decagon, etc. In some examples, the plurality of transducer controllers 206 may be used for controlling the plurality of ultrasound transducer elements 212 to obtain imaging data associated with the vessel 120.

The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014, ('220 Application). The support member 230 can be a ferrule having a distal flange or portion 232 and a proximal flange or portion 234. The support member 230 can be tubular in shape and define a lumen 236 extending longitudinally therethrough. The lumen 236 can be sized and shaped to receive the guide wire 118. The support member 230 can be manufactured using any suitable process. For example, the support member 230 can be machined and/or electrochemically machined or laser milled, such as by removing material from a blank to shape the support member 230, or molded, such as by an injection molding process.

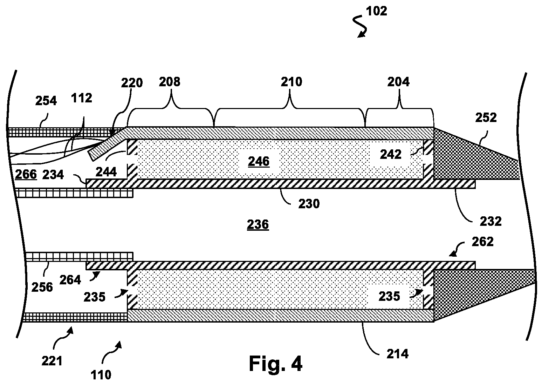

**Fig. 4** is a diagrammatic cross-sectional side view of a distal portion of the intraluminal imaging device 102, including the flexible substrate 214 and the support member 230, according to aspects of the present disclosure. The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014. The support member 230 can be a ferrule having a distal portion 262 and a proximal portion 264. The support member 230 can define a lumen 236 extending along the longitudinal axis LA. The lumen 236 is in communication with the entry/exit port 116 and is sized and shaped to receive the guide wire 118 (Fig. 1A). The support member 230 can be manufactured according to any suitable process. For example, the support member 230 can be machined and/or electrochemically machined or laser milled, such as by removing material from a blank to shape the support member 230, or molded, such as by an injection molding process. In some embodiments, the support member 230 may be integrally formed as a unitary structure, while in other embodiments the support member 230 may be formed of different components, such as a ferrule and stands 242, 244, that are fixedly coupled to one another. In some cases, the support member 230 and/or one or more components thereof may be completely integrated with inner member 256. In some cases, the inner member 256 and the support member 230 may be joined as one, e.g., in the case of a polymer support member.

Stands 242, 244 that extend vertically are provided at the distal and proximal portions 262, 264, respectively, of the support member 230. The stands 242, 244 elevate and support the distal and proximal portions of the flexible substrate 214. In that regard, portions of the flexible substrate 214, such as the transducer portion or region 204, can be spaced from a central body portion of the support member 230 extending between the stands 242, 244. The stands 242, 244 can have the same outer diameter or different outer diameters. For example, the distal stand 242 can have a larger or smaller outer diameter than the proximal stand 244 and can also have special features for rotational alignment as well as control chip placement and connection. To improve acoustic performance, any cavities between the flexible substrate 214 and the surface of the support member 230 are filled with a backing material 246. The liquid backing material 246 can be introduced between the flexible substrate 214 and the support member 230 via passageways 235 in the stands 242, 244. In some embodiments, suction can be applied via the passageways 235 of one of the stands 242, 244, while the liquid backing material 246 is fed between the flexible substrate 214 and the support member 230 via the passageways 235 of the other of the stands 242, 244. The backing material can be cured to allow it to solidify and set. In various embodiments, the support member 230 includes more than two stands 242, 244, only one of the stands 242, 244, or neither of the stands. In that regard the support member 230 can have an increased diameter distal portion 262 and/or increased diameter proximal portion 264 that is sized and shaped to elevate and support the distal and/or proximal portions of the flexible substrate 214.

The support member 230 can be substantially cylindrical in some embodiments. Other shapes of the support member 230 are also contemplated including geometrical, non-geometrical, symmetrical, non-symmetrical, cross-sectional profiles. As the term is used herein, the shape of the support member 230 may reference a cross-sectional profile of the support member 230. Different portions the support member 230 can be variously shaped in other embodiments. For example, the proximal portion 264 can have a larger outer diameter than the outer diameters of the distal portion 262 or a central portion extending between the distal and proximal portions 262, 264. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

A proximal inner member 256 and a proximal outer member 254 are coupled to the proximal portion 264 of the support member 230. The proximal inner member 256 and/or the proximal outer member 254 can include a flexible elongate member. The proximal inner member 256 can be received within a proximal flange 234. The proximal outer member 254 abuts and is in contact with the flexible substrate 214. A distal member 252 is coupled to the distal portion 262 of the support member 230. For example, the distal member 252 is positioned around the distal flange 232. The distal member 252 can abut and be in contact with the flexible substrate 214 and the stand 242. The distal member 252 can be the distal-most component of the intraluminal imaging device 102.

One or more adhesives can be disposed between various components at the distal portion of the intraluminal imaging device 102. For example, one or more of the flexible substrate 214, the support member 230, the distal member 252, the proximal inner member 256, and/or the proximal outer member 254 can be coupled to one another via an adhesive.

The conductor interface 220 is positioned at a proximal end of the substrate 214, and provides a point of electrical contact for the transmission line bundle 112. As described above, the transmission line bundle 112 may comprise a plurality of conductors configured to carry signals to and from the electrical components positioned on the substrate. The conductors of the transmission line bundle 112 are sized, shaped, and otherwise configured to be positioned within the space or lumen 266 between the proximal outer member 254 and the proximal inner member 256.

As described above, space available within the spaces provided in the elongate body of the catheter (e.g., within the proximal outer member 254) may be limited. One approach to positioning the conductors of the transmission line bundle 112 within the limited spaces of the catheter is to use a single group of small-gauge wires or ribbons spanning an entire length of the catheter from the scanner assembly to the PIM. The conductors of the bundle 112 may be bundled together to form one or more twisted pairs, twisted quads, twisted groups, or other arrangements of conductors. In some embodiments, one or more of the conductors is non-twisted, such that it runs parallel with one or more conductors or twisted groups of conductors.

It will be understood that, while the embodiments described below include IVUS imaging catheters, the present disclosure contemplates that the described structural features and/or arrangements may be used in other types of intraluminal devices, including sensing catheters, guide catheters, imaging probes, sensing probes, or any other suitable type of device.

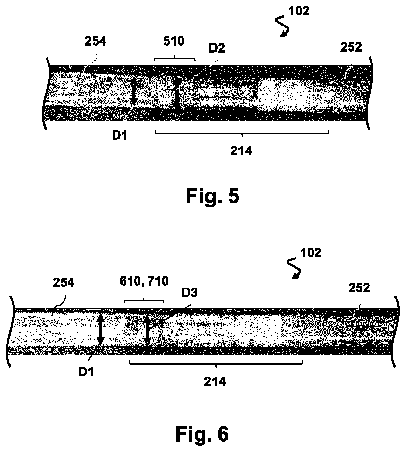

**Fig. 5** is a side view of a distal portion of the intraluminal imaging device 102, including the flexible substrate 214 and the outer member 254 with an adhesive joint 510 between them. Also visible is the distal member or distal tip 252. As can be seen in Figure 5, the diameter D2 of the adhesive joint 510 is slightly larger than the diameter D1 of the outer member 254, because layer(s) of adhesive are used to seal the joint between the outer member 254 and the substrate 214 (e.g., adhesive disposed between the outer member 254 and the flexible substrate 214 and adhesive around the outside of the outer member 254 and the flexible substrate 214). Assembling this structure requires the additional steps of introducing the adhesive to the joint and then curing the adhesive.

**Fig. 6** is a side view of a distal portion of the intraluminal imaging device 102, including the flexible substrate 214 and the outer member 254 with a heat seal joint 610 between them, in accordance with at least one embodiment of the present disclosure. Also visible is the distal member 252. As can be seen in Figure 6, the diameter D3 of the adhesive joint 510 is approximately the same as the diameter D1 of the outer member 254, because the outer member is positioned over a proximal portion of the flexible substrate 214 and then thermally reflowed, while temporarily enclosed within a heat-shrink outer mold tool, which is then removed after thermal reflow. In an example, the heat shrink outer mold tool is made of a polymer such as PTFE. This smaller diameter D3 advantageously makes the intraluminal device 102 easier to maneuver within a body lumen and is possible because layer(s) of adhesive need not be used at the joint, as compared to the diameter D2 of the adhesive joint 510 in Fig. 5. By avoiding the steps of introducing and curing the adhesive, the assembly process for the device 102 in Figure 6 advantageously takes less time and requires fewer steps than the assembly process for an adhesive joint 510 as shown in Fig. 5. The heat seal joint or thermally bonded imaging joint 610 thus formed in an overlap region 710 can form a portion of the exterior surface of the device 102 and/or couple the outer member 254 to other components of the device 102. For example, this advantageously seals and/or otherwise holds the components of the device 102 together by preventing them from detaching from one another, e.g., such as during use inside a patient's body. In some embodiments, the heat seal joint or thermally bonded imaging joint 610 thus formed in an overlap region 710 can resist fluid ingress and thus protect the electronics of the flexible substrate 214 from body fluids. The heat seal joint 610 also provides a flexible transition that may have a smaller diameter, be more maneuverable, be less prone to kinking, and be less labor intensive to fabricate. As described in more detail herein, the heat seal joint 610 can include a polymer filler member that is thermally bonded to other compatible polymer components of the device (e.g., the outer member 254 and/or an inner member).

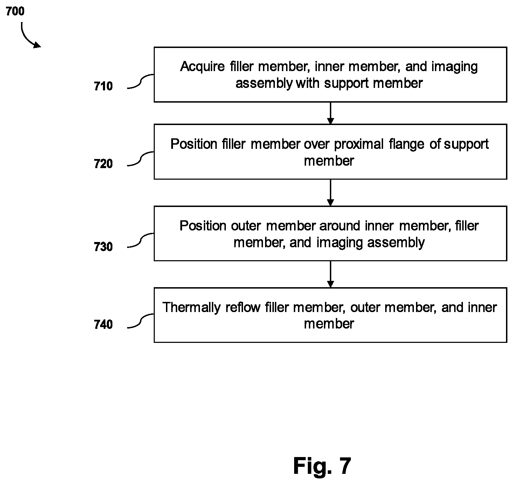

**Fig. 7** is a flow diagram 700 of an example assembly process for the heat seal joint 610 as described in Fig. 6, in accordance with at least one embodiment of the present disclosure. In step 710, an assembly technician, assembly robot, or other assembler acquires a filler member, an inner member (e.g., inner member 256 of Fig. 4), and an imaging assembly (e.g., imaging assembly 110 of Fig. 4) comprising a flexible substrate (e.g., flexible substrate 214 of Fig. 4) that has been wrapped around and coupled to a support member (e.g., support member 230 of Fig. 4). In an example, a distal end of the inner member is inserted into the proximal flange (e.g., proximal flange 234 of Fig. 4) of the support member.

In step 720, the assembler positions the filler member over the proximal flange, such that the filler member fits outside the distal portion of the inner member and at least a portion of the proximal flange.

In step 730, the assembler positions the outer member (e.g., outer member 254 of Fig. 4) over the inner member, and positions a distal end of the outer member over the filler member, support member, and a proximal portion of the imaging assembly (e.g., imaging assembly 110 of Fig. 4) or flexible substrate (e.g., flexible substrate 214 of Fig. 4). This forms an overlap region (e.g., region 710 of Fig. 6) where the filler member occupies the same longitudinal position along the flexible elongate member as a portion of the inner member, a portion of the outer member, a portion of the flexible substrate, and at least a portion of the proximal flange.

In step 740, the assembler applies heat to the overlap region (e.g., with a heat sealing fixture, while the flexible elongate member is held in place with by an assembly mandrel), at a temperature and heat flux sufficient to thermally reflow the inner member, outer member, and filler member within the overlap region. This permits the inner member, filler member, and outer member to form a thermal bond to one another within the overlap region, and also permits the inner member and filler member to thermally conform to the contours of the proximal flange of the support member, and permits the outer member to thermally conform to the contours of a proximal portion of the flexible substrate 214, even though the proximal flange and flexible substrate are not thermally reflowed. This heating and reflow process converts the overlap region into a thermal bond or heat seal joint (e.g., heat seal joint 610 of Fig. 6), which not only joints the inner member, filler member, and outer member together, but also forms a tight seal between the inner member and the proximal flange, and between the outer member and the flexible substrate. This heat seal joint advantageously resists fluid ingress, resists kinking, and/or presents a cross-sectional diameter similar to the diameter of the outer member proximal of the overlap region.

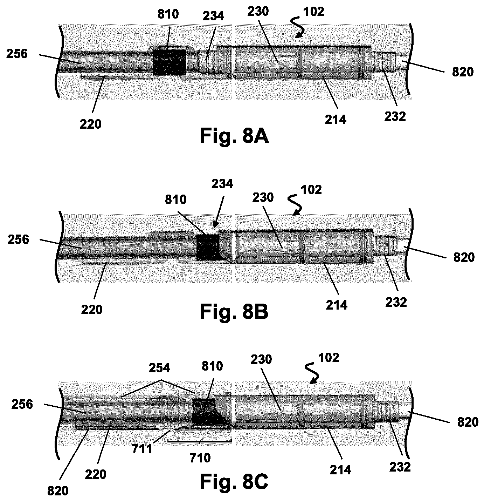

**Fig. 8A** is a side view of an intraluminal imaging device 102 during an assembly process, in accordance with at least one embodiment of the present disclosure. Visible are the inner member 256, weld leg or conductor interface 220, and support member 230, including a proximal flange 234 and distal flange 232. Also visible is the flexible substrate 214, which has been wrapped around, and adhered to, the support member 230, and an assembly mandrel 820 that has been inserted into the distal flange 232 to hold the intraluminal imaging device 102 in place during the assembly process. As part of the assembly process to create a heat seal joint, a filler member 810 is slid into place over the inner member 256 and moved proximally such that it slides between the inner member 256 and the weld leg or conductor interface 220. Examples of the weld leg 220 are described are disclosed in U.S. Patent Application No. 63/056,152, titled "CURVED CIRCUIT SUBSTRATE FOR INTRALUMINAL ULTRASOUND IMAGING ASSEMBLY" (Attorney Docket No.: 2020PF00249 / 44755.2148PV01) and filed July 24, 2020. For example, the conductor interface 220 may be implemented with an irregular shape and/or bend along a curved path.

**Fig. 8B** is a side view of an intraluminal imaging device 102 during an assembly process, in accordance with at least one embodiment of the present disclosure. Visible are the inner member 256, weld leg or conductor interface 220, support member 230, proximal flange 234, distal flange 232, flexible substrate 214, and assembly mandrel 820. As part of the assembly process, to create the heat seal joint, the filler member 810 is slid into place over the inner member 256 until the filler member 810 covers at least a portion of the proximal flange 234, while still overlapping at least partially with the inner member 256 past the proximal end of the proximal flange 234.

**Fig. 8C** is a side view of an intraluminal imaging device 102 during an assembly process, in accordance with at least one embodiment of the present disclosure. Visible are the inner member 256, weld leg or conductor interface 220, support member 230, proximal flange 234, distal flange 232, flexible substrate 214, and assembly mandrel 820. As part of the assembly process, to create the heat seal joint, the outer member 254 is slid into place over the inner member 256 and filler member 810, until the outer member 254 completely covers (or covers a majority of) the proximal flange 234 and filler member 810, and a portion of the flexible substrate 214, forming an overlap region 710 where the inner member 256, filler member 810, and outer member 254 all overlap. For example, the distal end of the outer member 254 can flared. This flared portion 711 of the outer member 254 can have an inner diameter and/or an outer diameter that larger than the inner diameter and/or the outer diameter of more proximal portions of the outer member. The flared portion 711 can be positioned over the proximal flange 234, the filler member 810, and a portion of the flexible substrate 214, to form the overlap region 710. At this point, heat can be applied to the overlap region 710 to form a heat seal joint (e.g., heat seal joint 610 of Fig. 6).

A proximal portion 820 of the conductor interface or weld leg 220 is configured to be mechanically and electrically coupled (e.g., by welding or soldering) to the conductors 218 of the cable 112. The proximal portion 820 of the conductor interface or weld leg 220 is positioned proximal of the proximal end of the filler member 810.

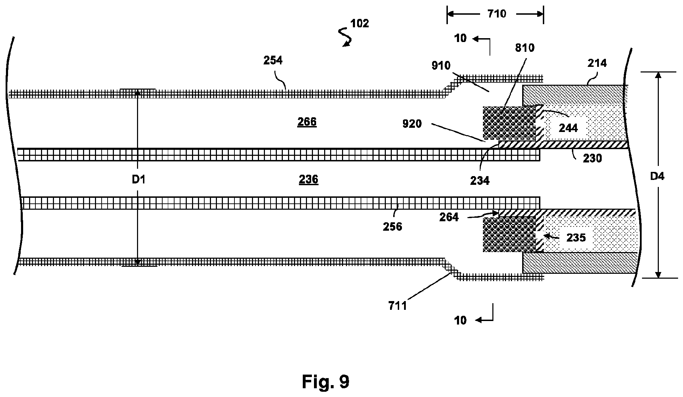

**Fig. 9** is a side cross-sectional view of an intraluminal imaging device 102 prior to the formation of the heat seal joint 610, in accordance with at least one embodiment of the present disclosure. Visible are the inner member 256, outer member 254, guidewire lumen 236, space 266, proximal flange 234 of support member 230, support member proximal stand 244, support member stand passageway 235, proximal portion 264 of support member 230, flexible substrate 214, flared portion 711 of the outer member 254, and filler member 810. The weld leg 220 and conductors 218, which occupy a portion of space 266, are not visible in Fig. 9. In that regard, the cross-sectional view of Fig. 9 can be taken along a different cross-sectional plane compared to the cross-sectional view of Fig. 4, which does include weld leg 220 and conductors 112. For example, the cross-sectional plane in Fig. 9 is rotationally offset relative to the cross-sectional plane in Fig. 4 (e.g., around a central longitudinal axis of the device 102). It is understood that the weld leg 220 and conductors 218 can be part of the device 102.

In an example, the filler member 810 is positioned over the proximal flange 234 of the support member 230, such that a distal end of the filler member 810 is proximate to, adjacent to, or in contact with the support member proximal stand 244. The proximal flange 234 defines the proximal end of the support member 230. At least a portion of the filler member 810 may be positioned proximal of the proximal end of the proximal flange 234, such that a portion of the filler member 810 surrounds the inner member 256 without the proximal flange 234 in between them. This arrangement permits thermal bonding between the filler member 810 and the inner member 256. For example, in the example shown in Fig. 9, the inner member 256, filler member 810, and outer member 254 overlap longitudinally in the overlap region 710. However, prior to thermal reflow, a gap or lumen 910 exists radially between the outer member 254 and the filler member 810 within the overlap region 710, and a gap or lumen 920 exists radially between the filler member 810 and the inner member 256 within the overlap region 710. In other words, the inner member 256 is positioned within the lumen 266 or 910 of the outer member 254 and the lumen 920 of the filler member 810. The flared portion 710, fitted around the flexible substrate 214, forms a diameter D4 that is greater than the diameter D1 of the outer member 254 proximal of the overlap region 710.

It is noted that when a guidewire 118 is extended through the guidewire lumen 236 of the inner member 256, it also passes through the lumen 920 of the filler member 810, and the lumen 266, 910 of the outer member 254.

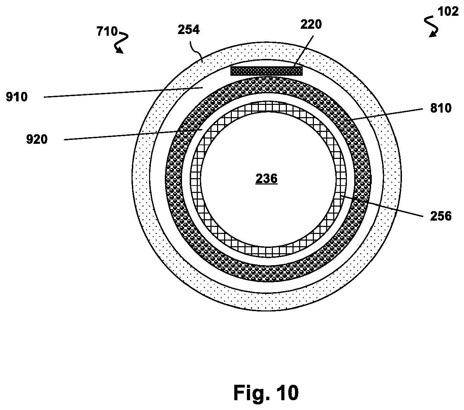

**Fig. 10** is a cross-sectional end view, cut along plane 10-10 of Fig. 9, of the overlap region 710 of an intraluminal imaging device 102 prior to the formation of the heat seal joint 610, in accordance with at least one embodiment of the present disclosure. Visible are the outer member 254, weld leg or conductor interface 220, filler member 810, inner member 256, and lumen 236. Prior to thermal reflow, a gap 910 exists radially between the outer member 254 and the filler member 810 within the overlap region 710, and a gap 920 exists radially between the filler member 810 and the inner member 256 within the overlap region 710.

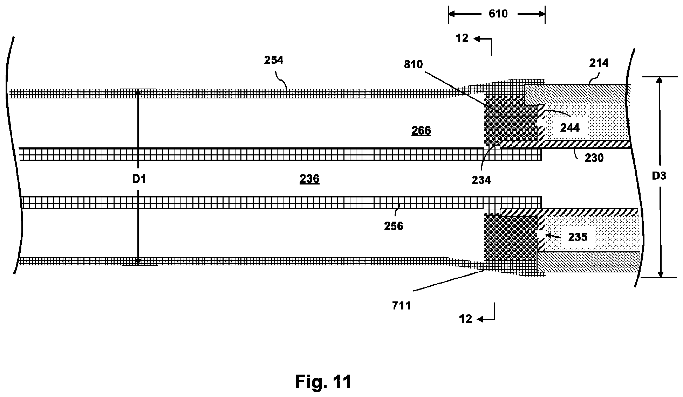

**Fig. 11** is a side cross-sectional view of an intraluminal imaging device 102 subsequent to the formation of the heat seal joint 610, in accordance with at least one embodiment of the present disclosure. Visible are the inner member 256, outer member 254, guidewire lumen 236, space 266, proximal flange 234 of support member 230, support member proximal stand 244, support member stand passageway 235, flexible substrate 214, flared portion 711 of the outer member 254, and filler member 810. The cross-sectional view in Fig. 11 is taken along the same cross-sectional plane as in Fig. 9, in which the weld leg 220 and conductors 218 are not visible.

The inner member 256, filler member 810, and outer member 254 overlap longitudinally in the thermal bond region or heat seal region 610. Subsequent to thermal reflow, no gaps exist within the heat seal region 610 between the outer member 254 and the filler member 810, because these elements have thermally reflowed, bonded, and intermingled with one another. Similarly, no gap exists between the filler member 810 and the inner member 256, because these elements have thermally reflowed, bonded, and intermingled with one another. This thermal reflow also causes the filler member 810 and inner member 256 to conform to the contours of the proximal flange 234, causing them to adhere to the proximal flange 234, and causes the outer member 254 and filler member 810 to conform to the contours of the flexible substrate 214, causing them to adhere to the flexible substrate 214. The resulting heat seal bond 610 resists kinking and/or fluid ingress, even as the intraluminal imaging device 102 is maneuvered through the tortuous pathways of a patient's body lumen. As a result of thermal reflow, the outer diameter of the flared portion 711 of the outer member 254 decreases from a value D4 (as shown in Fig. 9) to a smaller value D3. This is because the material is heated to the point at which is deforms and flows into the gaps 910, 920 that existed prior to reflow. Diameter D3 may be more similar (than diameter D4) to the diameter D1 of the outer member 254 proximal of the heat seal region 610. Advantageously, the decrease in the outer diameter of the flared portion 711 provides a smaller outer diameter for the device 102 with a thermally bonded joint 610, because there is no need for space radially inward of the outer member 254 to receive adhesive, as for an adhesive filled joint. Other changes in the dimensions of the outer member 254, filler member 810, and/or inner member 256 may also occur as a result of thermal reflow. For example, the outer diameter of the filler member 810 may decrease and the length of the filler member 810 may increase, as a result of flow of the heated polymer. In one non-limiting example, D1 is approximately 0.037"(0.94 mm), and D4 is less than approximately 0.415"(10.5 mm), while the outer diameter of the rolled-up flex circuit 214 is approximately 0.040"(1.0 mm).

In an example, the inner member, filler member, and outer member are all made of the same or similar polymers, such as polyether block amide (PEBAX), polytetrafluoroethylene (PTFE), or polyethylene (PE), or blends thereof. In other embodiments, the inner member, filler member, and outer member may all be made from different polymers, or else two may be made from the same or similar polymers, and a third made from a different polymer, so long as the polymer of the outer member 254 is capable of thermally bonding with the polymer of the filler member 810, and the polymer of the filler member 810 is capable of thermally bonding with the polymer of the inner member 256. Further, although the boundaries between elements 254, 810, and 256 are shown in Fig. 11 as being straight, it should be understood that after thermal reflow, the boundaries between these elements may be irregular, indistinct, or even undetectable.

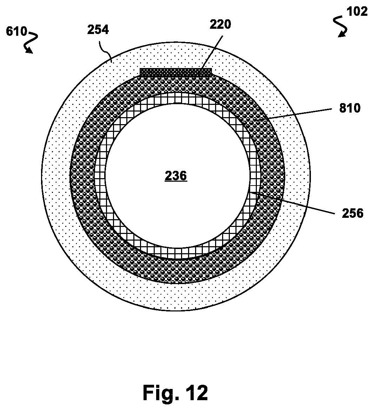

**Fig. 12** is a cross-sectional end view, cut along plane 12-12 of Fig. 11, of the heat seal region 610 of an intraluminal imaging device 102 subsequent to the formation of the heat seal joint 610, in accordance with at least one embodiment of the present disclosure. Visible are the outer member 254, weld leg or conductor interface 220, filler member 810, inner member 256, and lumen 236. Subsequent to thermal reflow, no gap exists between the outer member 254 and the filler member 810 within the heat seal region 610, and no gap 920 exists between the filler member 810 and the inner member 256 within the heat seal region 610, because the polymer materials of elements 256, 810, and 254 have reflowed, intermingled, and bonded to one another. Although the boundaries between elements 254, 810, and 256 are shown in Fig. 12 as being circular or approximately circular, it should be understood that after thermal reflow, the boundaries between these elements may be irregular, indistinct, or even undetectable. It is also noted that after thermal reflow, within at least a longitudinal portion of the heat seal region 610, the conductor interface or weld leg 220 is completely surrounded by at least one polymer, e.g., by the thermally reflowed polymer of the outer member 254, or the thermally reflowed polymer of the filler member 810, or the thermally reflowed polymer of inner member 256, or by the intermingled polymers of at least two of the outer member 254, filler member 810, and inner member 256. The conductor interface or weld leg 220 is proximate to the outer member 254, the filler member 810, and the inner member 256. The conductor interface or weld leg is positioned radially outward of the filler member. The outer member is positioned radially outward of the conductor interface. After reflow, the conductor interface is adjacent to the outer member and the filler member. In some embodiments, the conductor interface or weld leg 220 may be adjacent to two of the polymers (e.g., polymer 254 and polymer 810) such that the polymers completely surround the conductor interface or weld leg 220.

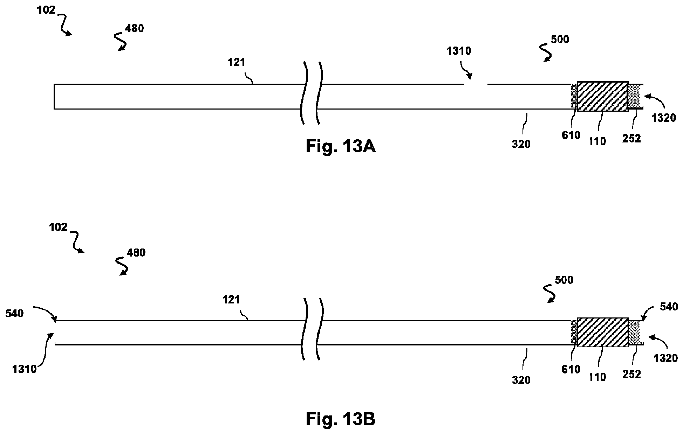

**Fig. 13A** is a diagrammatic, schematic view of an intraluminal imaging device 102 including a heat seal joint 610 and a flexible elongate member 121. The flexible elongate member 121 has a proximal portion 480 terminating at a proximal end, and a distal portion 500 terminating at a distal end. As further illustrated in Fig. 13A, in some embodiments, the flexible elongate member 121 may be a rapid-exchange catheter. To that end, the flexible elongate member 121 may include a guidewire entry port 1310 and a guidewire exit port 1320 positioned in the distal portion 500 of the flexible elongate member. In some cases, the guidewire exit port 1320 may be at a distal end of the distal member 252, and the heat seal joint 610 may be located at a proximal end of the scanner assembly 110, distal of the guidewire entry port 1310 and proximal of the guidewire exit port 1320.

**Fig. 13B** is a diagrammatic schematic view of an example intraluminal imaging device 102, wherein the flexible elongate member 121 is implemented as an over-the-wire catheter. More specifically, in the illustrated example, the guidewire entry port 1310 is positioned at a proximal end 540 of the flexible elongate member (e.g., within the proximal portion 480). In this way, the flexible elongate member 121 may be implemented so that the flexible elongate member 121 can extend over a guide wire (e.g., guide wire 118) from a proximal end 540 to a distal end 550 of the flexible elongate member. As further illustrated by Fig. 13B, in embodiments where the flexible elongate member 121 is an over-the-wire catheter, the heat seal joint 610 may be positioned in the distal portion 500 of the flexible elongate member, at a proximal end of the scanner assembly 110. In other embodiments, other types of flexible elongate members 121 may be used, instead of or in addition to the types shown in Figs. 13A and 13B.

A person of ordinary skill in the art will recognize that the present disclosure advantageously provides an intraluminal imaging system that enables both pushability and trackability for navigating an imaging assembly through human vasculature, while providing a seal between the flexible elongate member 121 and the scanner assembly 110 that resists kinking and/or fluid ingress. Trackability may refer, for example, to how well a device can be accurately and smoothly navigated through lengths of vasculature (e.g., the flexible elongate member's ability to follow the tip along a vessel), including tortuous pathways, bends, stenoses, intersections, confluences, etc. The logical operations making up the embodiments of the technology described herein may be referred to variously as operations, steps, objects, elements, components, regions, etc. Furthermore, it should be understood that these may occur or be arranged in any order, unless explicitly claimed otherwise or a specific order is inherently necessitated by the claim language.

It should further be understood that the described technology may be employed in a variety of different applications, including but not limited to human medicine, veterinary medicine, education and inspection. All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the intraluminal imaging system. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. Accordingly, it is appropriate that the appended claims be construed broadly and in a manner consistent with the present disclosure.

## Claims

1. An intraluminal imaging device (102), comprising:
a flexible elongate member (121) configured to be positioned within a body lumen of a patient, wherein the flexible elongate member comprises a first polymer;
a scanner assembly (200) configured to obtain imaging data while positioned within the body lumen, wherein the scanner assembly is positioned at a distal end of the flexible elongate member; and
a filler member (810) comprising a second polymer and positioned at the distal end of the flexible elongate member, wherein the filler member is coupled to the flexible elongate member via thermal reflow of the first polymer and the second polymer to seal a joint (610) between the flexible elongate member and the scanner assembly, wherein the filler member comprises a lumen (920), wherein the flexible elongate member comprises:
an outer member (254) comprising a lumen (266); and
an inner member ( 256) extending within the lumen (266) of the outer member and the lumen (920) of the filler member; and
wherein the outer member comprises the first polymer,
wherein the inner member comprises a third polymer, and
wherein the filler member is coupled to the flexible elongate member via thermal reflow of the second polymer and the third polymer.

2. The intraluminal imaging device of claim **1,** wherein the first polymer, the second polymer and the third polymer are the same such as polyether block amide, polytetrafluoroethylene, or polyethylene, or blends thereof.

3. The intraluminal imaging device of claim 1, wherein two of the first polymer, the second polymer and the third polymer are the same and a third of the first polymer, the second polymer and the third polymer is different from the other two.

4. The device of claim 1,
wherein the inner member (256) comprises a lumen (236) configured to receive a guidewire, and
wherein the filler member is arranged such that, when the guidewire is received within the lumen of the inner member, the guidewire extends within the lumen of the filler member.

5. The device of claim 1, wherein the filler member is positioned at a proximal portion of the scanner assembly.

6. The device of claim 5, wherein the distal end of the flexible elongate member is positioned over the proximal portion of the scanner assembly and the filler member.

7. The device of claim 6,
wherein the distal end of the flexible elongate member comprises a flared portion (711) of the outer member, and
wherein the flared portion is coupled to the filler member.

8. The device of claim 1,
wherein the scanner assembly comprises a support member (230) and an array of acoustic elements (212) positioned around the support member, and
wherein the filler member is positioned around a proximal portion of the support member.

9. The device of claim 8,
wherein the proximal portion of the support member comprises a proximal flange (234) defining a proximal end of the support member and configured to receive the inner member, and
wherein the filler member is positioned around the proximal flange.

10. The device of claim 9,
wherein the proximal portion of the support member comprises a proximal stand (244),
wherein a diameter of the proximal stand is greater than a diameter of the proximal flange, and
wherein the filler member is positioned adjacent to the proximal stand.

11. The device of claim 9,
wherein the proximal portion of the support member terminates at a proximal end of the support member, and
wherein the proximal end of the filler member is positioned proximal of the proximal end of the support member.

12. The device of claim 1, wherein the scanner assembly is an ultrasound scanner assembly, the device further comprising:
a plurality of conductors (218) in communication with the ultrasound scanner assembly and extending along the flexible elongate member,
wherein the ultrasound scanner assembly comprises a conductor interface (220),
wherein the plurality of conductors are coupled to the conductor interface, and
wherein the conductor interface is adjacent to at least one of the first polymer or the second polymer.

13. The device of claim 12,
wherein the plurality of conductors are coupled to the conductor interface at a proximal portion of the conductor interface, and
wherein the proximal portion of the conductor interface is positioned proximal of the filler member.

14. The device of claim 1,
wherein the flexible elongate member comprises a rapid-exchange catheter with a guidewire entry port,
wherein the guidewire entry port is disposed at a distal portion of the flexible elongate member, and
wherein the filler member is positioned distal of the guidewire entry port.

15. The intraluminal imaging device of claim 1, wherein:
the intraluminal imaging is intravascular ultrasound (IVUS) imaging;
the device is a catheter;
the flexible elongate member is a catheter body;
the body lumen is a blood vessel;
the scanner assembly is an ultrasound scanner assembly and the imaging data are ultrasound imaging data;
the ultrasound scanner assembly comprises a circumferential array of acoustic elements configured to obtain the ultrasound imaging data;
wherein the filler member is positioned at a proximal portion of the ultrasound scanner assembly; and
wherein a distal end of the outer member is positioned around the proximal portion of the ultrasound scanner assembly and the filler member.

16. An assembly process for an intraluminal imaging device of any one of claims 1 to 14, the process comprising:
acquiring a filler member, an inner member and an imaging assembly with support member;
position the filler member over a proximal flange of the support member;
position the outer member around the inner member, the filler member and the imaging assembly; and
thermally reflow the filler member, the outer member and the inner member.

## Patentansprüche

1. Intraluminale Bildgebungsvorrichtung (102), umfassend:
ein flexibles, längliches Element (121), das so konfiguriert ist, dass es innerhalb eines Körperlumens eines Patienten positioniert werden kann, wobei das flexible, längliche Element ein erstes Polymer umfasst;
eine Scanneranordnung (200), die so konfiguriert ist, dass sie Bilddaten erhält, während sie innerhalb des Körperlumens positioniert ist, wobei die Scanneranordnung an einem distalen Ende des flexiblen, länglichen Elements positioniert ist; und
ein Füllelement (810), das ein zweites Polymer umfasst und am distalen Ende des flexiblen, länglichen Elements positioniert ist, wobei das Füllelement durch thermischen Rückfluss des ersten Polymers und des zweiten Polymers mit dem flexiblen, länglichen Element gekoppelt ist, um eine Verbindung (610) zwischen dem flexiblen, länglichen Element und der Scanneranordnung abzudichten, wobei das Füllelement ein Lumen (920) umfasst, wobei das flexible, längliche Element umfasst:
ein äußeres Element (254), das ein Lumen (266) umfasst; und
ein inneres Element (256), das sich innerhalb des Lumens (266) des äußeren Elements und des Lumens (920) des Füllelements erstreckt; und
wobei das äußere Element das erste Polymer umfasst,
wobei das innere Element ein drittes Polymer umfasst, und
wobei das Füllelement durch thermischen Rückfluss des zweiten Polymers und des dritten Polymers mit dem flexiblen, länglichen Element gekoppelt ist.

2. Intraluminale Bildgebungsvorrichtung nach Anspruch 1, wobei das erste Polymer, das zweite Polymer und das dritte Polymer gleich sind, beispielsweise Polyetherblockamid, Polytetrafluorethylen oder Polyethylen oder Mischungen davon.

3. Intraluminale Bildgebungsvorrichtung nach Anspruch 1, wobei zwei des ersten Polymers, des zweiten Polymers und des dritten Polymers gleich sind und ein Drittel des ersten Polymers, des zweiten Polymers und des dritten Polymers sich von den anderen zwei unterscheidet.

4. Vorrichtung nach Anspruch 1,
wobei das innere Element (256) ein Lumen (236) umfasst, das zur Aufnahme eines Führungsdrahtes konfiguriert ist, und
wobei das Füllelement so angeordnet ist, dass der Führungsdraht, wenn der Führungsdraht innerhalb des Lumens des inneren Elements aufgenommen wird, sich innerhalb des Lumens des Füllelements hinein erstreckt.

5. Vorrichtung nach Anspruch 1, wobei das Füllelement an einem proximalen Abschnitt der Scanneranordnung positioniiert ist.

6. Vorrichtung nach Anspruch 5, wobei das distale Ende des flexiblen, länglichen Elements über dem proximalen Abschnitt der Scanneranordnung und dem Füllelement positioniert ist.

7. Vorrichtung nach Anspruch 6,
wobei das distale Ende des flexiblen, länglichen Elements einen aufgeweiteten Abschnitt (711) des äußeren Elements umfasst, und
wobei der aufgeweitete Abschnitt mit dem Füllelement gekoppelt ist.

8. Vorrichtung nach Anspruch 1,
wobei die Scanneranordnung ein Stützelement (230) und eine um das Stützelement herum positionierte Anordnung akustischer Elemente (212) umfasst, und
wobei das Füllelement um einen proximalen Abschnitt des Stützelements herum positioniert ist.

9. Vorrichtung nach Anspruch 8,
wobei der proximale Abschnitt des Stützelements einen proximalen Flansch (234) umfasst, der ein proximales Ende des Stützelements definiert und zur Aufnahme des inneren Elements konfiguriert ist, und
wobei das Füllelement um den proximalen Flansch herum positioniert ist.

10. Vorrichtung nach Anspruch 9,
wobei der proximale Abschnitt des Stützelements einen proximalen Ständer (244) umfasst, wobei ein Durchmesser des proximalen Ständers größer ist als der Durchmesser des proximalen Flansches, und
wobei das Füllelement angrenzend an den proximalen Ständer positioniert ist.

11. Vorrichtung nach Anspruch 9,
wobei der proximale Abschnitt des Stützelements an einem proximalen Ende des Stützelements endet, und
wobei das proximale Ende des Füllelements proximal zum proximalen Ende des Stützelements positioniert ist.

12. Vorrichtung nach Anspruch 1, wobei es sich bei der Scanneranordnung um eine Ultraschallscanneranordnung handelt, wobei die Vorrichtung weiter umfasst:
eine Vielzahl von Leitern (218), die mit der Ultraschallscanneranordnung in Kommunikation stehen und sich entlang des flexiblen, länglichen Elements erstrecken,
wobei die Ultraschallscanneranordnung eine Leiterschnittstelle (220) umfasst,
wobei die Vielzahl der Leiter mit der Leiterschnittstelle gekoppelt ist, und
wobei die Leiterschnittstelle an mindestens eines des ersten Polymers und des zweiten Polymers angrenzt.

13. Vorrichtung nach Anspruch 12,
wobei die Vielzahl von Leitern an einem proximalen Abschnitt der Leiterschnittstelle mit der Leiterschnittstelle gekoppelt ist, und
wobei der proximale Abschnitt der Leiterschnittstelle proximal zum Füllelement positioniert ist.

14. Vorrichtung nach Anspruch 1,
wobei das flexible, längliche Element einen Schnellwechselkatheter mit einer Einführungsöffnung für einen Führungsdraht umfasst,
wobei die Einführungsöffnung für den Führungsdraht an einem distalen Abschnitt des flexiblen, länglichen Elements angeordnet ist, und
wobei das Füllelement distal von der Einführungsöffnung des Führungsdrahtes positioniert ist.

15. Intraluminale Bildgebungsvorrichtung nach Anspruch 1, wobei:
die intraluminale Bildgebung intravaskuläre Ultraschallbildgebung (IVUS) ist;
die Vorrichtung ein Katheter ist;
das flexible, längliche Element ein Katheterkörper ist;
das Körperlumen ein Blutgefäß ist;
die Scanneranordnung eine Ultraschallscanneranordnung ist und die Bilddaten Ultraschallbilddaten sind;
die Ultraschallscanneranordnung eine umlaufende Anordnung akustischer Elemente umfasst, die so konfiguriert sind, dass sie die Ultraschallbilddaten erhalten;
wobei das Füllelement an einem proximalen Abschnitt der Ultraschallscanneranordnung positioniert ist; und
wobei ein distales Ende des äußeren Elements um den proximalen Abschnitt der Ultraschallscanneranordnung und des Füllelements herum positioniert ist.

16. Anordnungsprozess für eine intraluminale Bildgebungsvorrichtung nach einem der Ansprüche 1 bis 14, wobei das Verfahren umfasst:
Erfassen eines Füllelements, eines inneren Elements und einer Bildgebungsanordnung mit Stützelement;
Positionieren des Füllelements über einem proximalen Flansch des Stützelements;
Positionieren des äußeren Elements um das innere Element, das Füllelement und die Bildgebungsanordnung herum; und
thermischer Rückfluss des Füllelements, des äußeren Elements und des inneren Elements.

## Revendications

1. Dispositif d'imagerie intraluminale (102), comprenant :
un organe allongé flexible (121) configuré pour être positionné dans une lumière corporelle d'un patient, dans lequel l'organe allongé flexible comprend un premier polymère ;
un ensemble scanner (200) configuré pour obtenir des données d'imagerie lorsqu'il est positionné à l'intérieur de la lumière corporelle, dans lequel l'ensemble scanner est positionné au niveau d'une extrémité distale de l'organe allongé flexible ; et
un organe de remplissage (810) comprenant un deuxième polymère et positionné au niveau de l'extrémité distale de l'organe allongé flexible, dans lequel l'organe de remplissage est couplé à l'organe allongé flexible par refusion thermique du premier polymère et du deuxième polymère afin de sceller un joint (610) entre l'organe allongé flexible et l'ensemble scanner, dans lequel l'organe de remplissage comprend une lumière (920), dans lequel l'organe allongé flexible comprend :
un organe extérieur (254) comprenant une lumière (266) ; et
un organe intérieur (256) s'étendant à l'intérieur de la lumière (266) de l'organe extérieur et de la lumière (920) de l'organe de remplissage ; et
dans lequel l'organe extérieur comprend le premier polymère,
dans lequel l'organe intérieur comprend un troisième polymère, et
dans lequel l'organe de remplissage est couplé à l'organe allongé flexible par refusion thermique du deuxième polymère et du troisième polymère.

2. Dispositif d'imagerie intraluminale selon la revendication 1, dans lequel le premier polymère, le deuxième polymère et le troisième polymère sont identiques, tels que le polyéther bloc amide, le polytétrafluoroéthylène ou le polyéthylène, ou des mélanges de ceux-ci.

3. Dispositif d'imagerie intraluminale selon la revendication 1, dans lequel deux des premier, deuxième et troisième polymères sont identiques et un troisième des premier, deuxième et troisième polymères est différent des deux autres.

4. Dispositif selon la revendication 1,
dans lequel l'organe intérieur (256) comprend une lumière (236) configurée pour recevoir un fil-guide, et
dans lequel l'organe de remplissage est agencé de telle sorte que, lorsque le fil-guide est reçu dans la lumière de l'organe intérieur, le fil-guide s'étende dans la lumière de l'organe de remplissage.

5. Dispositif selon la revendication 1, dans lequel l'organe de remplissage est positionné dans une partie proximale de l'ensemble scanner.

6. Dispositif selon la revendication 5, dans lequel l'extrémité distale de l'organe allongé flexible est positionnée au-dessus de la partie proximale de l'ensemble scanner et de l'organe de remplissage.

7. Dispositif selon la revendication 6,
dans lequel l'extrémité distale de l'organe allongé flexible comprend une partie évasée (711) de l'organe extérieur, et
dans lequel la partie évasée est raccordée à l'organe de remplissage.

8. Dispositif selon la revendication 1,
dans lequel l'ensemble scanner comprend un organe de support (230) et un réseau d'éléments acoustiques (212) positionnés autour de l'organe de support, et
dans lequel l'organe de remplissage est positionné autour d'une partie proximale de l'organe de support.

9. Dispositif selon la revendication 8,
dans lequel la partie proximale de l'organe de support comprend une bride proximale (234) définissant une extrémité proximale de l'organe de support et configurée pour recevoir l'organe intérieur, et
dans lequel l'organe de remplissage est positionné autour de la bride proximale.

10. Dispositif selon la revendication 9,
dans lequel la partie proximale de l'organe de support comprend un socle proximal (244), dans lequel un diamètre du socle proximal est supérieur au diamètre de la bride proximale, et
dans lequel l'organe de remplissage est positionné adjacent au socle proximal.

11. Dispositif selon la revendication 9,
dans lequel la partie proximale de l'organe de support se termine au niveau d'une extrémité proximale de l'organe de support, et
dans lequel l'extrémité proximale de l'organe de remplissage est positionnée à proximité de l'extrémité proximale de l'organe de support.

12. Dispositif selon la revendication 1, dans lequel l'ensemble scanner est un ensemble scanner ultrasonore, le dispositif comprenant en outre :
une pluralité de conducteurs (218) en communication avec l'ensemble scanner ultrasonore et s'étendant le long de l'organe allongé flexible,
dans lequel l'ensemble scanner ultrasonore comprend une interface de conducteurs (220),
dans lequel la pluralité de conducteurs est couplée à l'interface de conducteurs, et
dans lequel l'interface de conducteurs est adjacente à au moins l'un des premier et deuxième polymères.

13. Dispositif selon la revendication 12,
dans lequel la pluralité de conducteurs sont couplés à l'interface de conducteurs au niveau d'une partie proximale de l'interface de conducteurs, et
dans lequel la partie proximale de l'interface de conducteurs est positionnée à proximité de l'organe de remplissage.

14. Dispositif selon la revendication 1,
dans lequel l'organe allongé flexible comprend un cathéter à échange rapide muni d'un orifice d'entrée de fil-guide,
dans lequel l'orifice d'entrée de fil-guide est disposé au niveau d'une partie distale de l'organe allongé flexible, et
dans lequel l'organe de remplissage est positionné à distance de l'orifice d'entrée de fil-guide.

15. Dispositif d'imagerie intraluminale selon la revendication 1, dans lequel :
l'imagerie intraluminale est une imagerie ultrasonore intravasculaire (IVUS) ;
le dispositif est un cathéter ;
l'organe allongé flexible est un corps de cathéter ;
la lumière corporelle est un vaisseau sanguin ;
l'ensemble scanner est un ensemble scanner ultrasonore et les données d'imagerie sont des données d'imagerie ultrasonore ;
L'ensemble scanner ultrasonore comprend un réseau circonférentiel d'éléments acoustiques configurés pour obtenir les données d'imagerie ultrasonore ;
dans lequel l'organe de remplissage est positionné au niveau d'une partie proximale de l'ensemble scanner ultrasonore ; et
dans lequel une extrémité distale de l'organe extérieur est positionnée autour de la partie proximale de l'ensemble scanner ultrasonore et de l'organe de remplissage.

16. Processus d'assemblage d'un dispositif d'imagerie intraluminale selon l'une quelconque des revendications 1 à 14, le processus comprenant :
l'acquisition d'un organe de remplissage, d'un organe intérieur et d'un ensemble d'imagerie avec un organe de support ;
le positionnement de l'organe de remplissage sur une bride proximale de l'organe de support ;
le positionnement de l'organe extérieur autour de l'organe intérieur, de l'organe de remplissage et de l'ensemble d'imagerie ; et
la refusion thermique de l'organe de remplissage, de l'organe extérieur et de l'organe intérieur.
